# EUROPÄISCHE PATENTSCHRIFT

(11) **EP 0 472 033 B1**
(45) Veröffentlichungstag und Bekanntmachung des Hinweises auf die Patenterteilung: **20.12.1995**
(21) Anmeldenummer: 91113017.7
(22) Anmeldetag: 02.08.1991
(51) Int. Cl.: A61K 31/195

(54) **3,5-Diiod-L-Thyronin zur TSH-Suppression und Behandlung von Struma**
3,5-Diiod-L-thyronin for TSH-suppression and the treatment of goiter
3,5-diiodo-L-thyronine pour la suppression de TSH et le traitement du goitre

(30) Priorität: 23.08.1990 DE 4026600
(43) Veröffentlichungstag der Anmeldung: 26.02.1992
(73) Patentinhaber: Henning Berlin Anlagen GmbH, D-12099 Berlin (DE)
(72) Erfinder: Horst, Claus, Dr. rer. nat. Dipl.-Biologe, W-1000 Berlin 42 (DE)
(74) Vertreter: Werner, Hans-Karsten, Dr.Dipl.-Chem.

(56) Entgegenhaltungen:
- ACTA ENDOCRINOL., Band 102, Suppl. 253, 1983; G. HINTZE et al., Seite 130, Nr. 144
- ENDOCRINOLOGY, Band 64, Nr. 1, 1959; N.R. STASILLI et al., Seiten 62-82
- INT. CONGRESS ON SER.-EXCERPTA MED., Band 403, ENDOCRINOLOGY, Band 2, 1977; E.C. JORGENSEN et al., Seiten 117-120
- BIOCHEMICAL JOURNAL, Band 261, Nr. 3, 1989, GB; C. HORST et al., Seiten 945-950

## Beschreibung

Eingehende vergleichende Untersuchungen der Wirksamkeit von Thyroxin und Thyroxin-Analogen in Ratten hatten ergeben, daß vor allem das um einen Iodatom ärmere 3,5,3'-Triiod-L-Thyronin (T₃) eine höhere Wirksamkeit besitzt als Thyroxin selbst. Alle übrigen Derivate waren deutlich schwächer wirksam; vergl. Neil R. Stasilli, ENDOCR. 64, Seiten 62 bis 82 (1959). Beim Vergleich der Wirksamkeit war insbesondere festgestellt worden, daß alle 3-Monoiod- und 3,5-Diiod-Verbindungen, die nicht auch in der 3'- und/oder 5'-Position mit Iod substituiert waren, unwirksam waren. Die einzige Ausnahme bildet das 3,5-Diiod-L-Thyronin (T₂), welches noch eine schwache Aktivität zeigt; vergl. Tabelle 1, Nr. 38, Seite 68, Absatz 2 und Seite 80, Absatz 2. Diese Ergebnisse sind später mehrfach bestätigt worden; vergl. Eugene C. Jorgensen in Hormonal Proteins and Peptides (ed: Li CH), Volume 6, Acad. Press New York 1978 in Tabelle III auf Seiten 132 und 133. Neuere vergleichende Untersuchungen von Thyroxin, Triiodthyronin und 3,5-Diiodthyronin bezüglich des Sauerstoffverbrauchs der Leber hypothyreoter Ratten haben gezeigt, daß 3,5-Diiod-L-Thyronin geringfügig rascher stimuliert und daß 3,5-Diiod-L-Thyronin diesbezüglich das wirksame Prinzip ist; vergl. Claus Horst et al., Biochem. J. (1989) 261, Seiten 945 bis 950. 3,5-Diiod-L-Thyronin ist somit als ein Metabolit der höher iodierten und hochwirksamen Substanzen anzusehen, welcher einen direkten Einfluß hat auf die Aktivität der Mitochondrien. Dabei ist jedoch zu beachten, daß 3,5-Diiod-L-Thyronin sehr rasch wirkt, während T₃ einen Langzeiteffekt besitzt.

Im Rahmen intensiver vergleichender Untersuchungen hochwirksamer Derivate von Triiod-Thyronin auf Stoffwechselparameter wie freie Fettsäuren, Triglyceride und Glukose wurde als Vergleichssubstanz auch 3,5-Diiod-L-Thyronin mituntersucht und dabei bestätigt, daß kaum eine signifikante Wirksamkeit besteht. Überraschenderweise wurde aber festgestellt, daß 3,5-Diiod-L-Thyronin bei langfristiger Applikation über Wochen in hochsignifikanter Weise den TSH-Spiegel senkt und sich damit als hochwirksames Mittel zur Behandlung von Struma eignet. Offensichtlich ist diese hohe Wirksamkeit des 3,5-Diiod-L-Thyronins in der Vergangenheit unerkannt geblieben, weil es diese Wirksamkeit erst zeigt, wenn es über längere Zeit appliziert wird. Die früheren Versuche waren demnach zu kurzfristig angelegt. Weiterhin wurde nicht TSH gemessen, sondern die Wirkung der einzelnen Substanzen auf die Rückbildung eines vorher provozierten Kropfes beurteilt. Zu der Fehlbeurteilung hat sicherlich beigetragen, daß 3,5-Diiod-L-Thyronin auch unwirksam ist bezüglich anderer Parameter, nämlich bezüglich des für die Schilddrüsenhormonwirkung klassischen Enzyms Glycerin-l-phosphatdehydrogenase sowie die Bindung an Zellkerne; vergl. Leeson et al., 1988, J. med. chem. 31, Seiten 37 bis 54. Da 3,5-Diiod-L-Thyronin in niedriger Dosierung praktisch keine Wirkung auf den Stoffwechsel hat, ist es ausgezeichnet geeignet zur TSH-Suppression und Behandlung von Struma. Die Dosierung kann daher in vergleichbarer Größenordnung gewählt werden wie für Thyroxin, ohne jedoch die damit verbundenen Nebenwirkungen aufzuweisen.

Gegenstand der vorliegenden Erfindung ist somit zunächst ein Mittel zur TSH-Suppression und Behandlung von Struma enthaltend eine therapeutisch wirksame Dosis 3,5-Diiod-L-Thyronin und übliche galenische Hilfsstoffe in Form von Tabletten, Dragees, Weichgelatinekapseln, Hartgelatinekapseln, Tropfen und Sirup. Vorzugsweise enthält dieses Mittel pro Dosiseinheit 1 bis 5 mg des Wirkstoffs 3,5-Diiod-L-Thyronin.

Weiterhin betrifft die Erfindung die Verwendung von 3,5-Diiod-L-Thyronin zur Herstellung eines Mittels zur TSH-Suppression und Behandlung von Struma.

Schließlich ist Gegenstand der vorliegenden Erfindung das Verfahren zur TSH-Suppression und Behandlung von Struma durch Applikation therapeutisch wirksamer Dosen von 3,5-Diiod-Thyronin.

Als Applikationsformen kommen vor allem Tabletten, Dragees, aber auch Tropfen und Sirup in Frage. Schließlich kann der Wirkstoff in Form von Weichgelatinekapseln oder Hartgelatinekapseln appliziert werden. Wichtig ist, daß jeweils eine therapeutisch wirksame Dosis zur Anwendung kommt. Diese Dosis kann aufgrund der Langzeitwirkung täglich auf einmal gegeben werden. Gewünschtenfalls kann aber auch die übliche Standarddosierung von dreimal täglich eine Dosiseinheit zur Anwendung kommen. Eine Dosiseinheit sollte somit 1 bis 5 mg 3,5-Diiod-L-Thyronin enthalten.

Aus den nachfolgenden Versuchsergebnissen geht hervor, in welchem Maße 3,5-Diiod-L-Thyronin eine TSH-Suppression bewirkt. Dazu wurden euthyreote Ratten, hypothyreote Ratten und adipöse Ratten 14 Tage bis 3 Wochen mit 3,5-Diiod-L-Thyronin behandelt und neben anderen Parametern der TSH-Spiegel gemessen. Dabei wurden folgende Werte gefunden:

| | | |
|---|---|---|
| Euthyreote Ratten (14d) | Kontrolle (NaCl) | 2,04 ng/ml |
| | 10 »g/100 g KG: | 1,42 ng/ml |
| | 100 »g/100 g KG: | 0,88 ng/ml |
| Hypothyreote Ratten (14d) | Kontrolle (NaCl) | 25,70 ng/ml |
| | 100 »g/100 g KG: | 3,20 ng/ml |
| Adipöse Ratten (21d) | Kontrolle (NaCl) | 4,00 ng/ml |
| | 10 »g/100 g KG: | 2,78 ng/ml |
| | 100 »g/100 g KG: | 2,51 ng/ml. |

Zum Vergleich wurden 15 »g Triiod-Thyronin (T₃) appliziert. Dabei sank der TSH-Spiegel bei euthyreoten Ratten auf 0,67 und bei hypothyreoten Ratten auf 3,40 ng/ml. Die Messung des Thyroxin-Serumspiegels ergab eine entsprechende Senkung.

Bei diesen Dosierungen wurde keine Veränderung des Glukose-Spiegels festgestellt und eine bisher nicht signifikante Verschiebung von Triglyceriden zu freien Fettsäuren.

## Patentansprüche

1. Mittel zur TSH-Suppression und Behandlung von Struma enthaltend eine therapeutisch wirksame Dosis 3,5-Diiod-L-Thyronin und übliche galenische Hilfsstoffe in Form von Tabletten, Dragees, Weichgelatinekapseln, Hartgelatinekapseln, Tropfen und Sirup.

2. Mittel gemäß Anspruch 1 enthaltend 1 bis 5 mg 3,5-Diiod-L-Thyronin pro Dosiseinheit.

3. Verwendung von 3,5-Diiod-L-Thyronin zur Herstellung eines Mittels zur TSH-Suppression und Behandlung von Struma in Form von Tabletten, Dragees, Weichgelatinekapseln, Hartgelatinekapseln, Tropfen und Sirup.

## Claims

1. An agent for TSH suppression and treatment of goiter containing a therapeutically effective amount of 3,5-diiodo-L-thyronine along with conventional galenic additives and being in the form of tablets, coated tablets, soft gelatin capsules, hard gelatin capsules, drops and syrups.

2. The agent according to claim 1 which contains from 1 to 5 mg of 3,5-diiodo-L-thyronine per dosage unit.

3. The use of 3,5-diiodo-L-thyronine for the preparation of an agent for TSH suppression and treatment of goiter in the form of tablets, coated tablets, soft gelatin capsules, hard gelatin capsules, drops and syrups.

## Revendications

1. Agent de freinage de la TSH et de traitement du goitre, contenant une dose thérapeutiquement efficace de 3,5-diiodo-L-thyronine et des adjuvants galéniques usuels sous forme de comprimés, de comprimés dragéifiés, de capsules molles, de gélules, de gouttes et de sirop.

2. Agent selon la revendication 1, contenant 1 à 5 mg de 3,5-diiodo-L-thyronine par unité de prise.

3. Utilisation de 3,5-diiodo-L-thyronine pour la préparation d'un agent de freinage de la TSH et de traitement du goitre sous forme de comprimés, comprimés dragéifiés, capsules molles, gélules, gouttes et sirop.
